# EUROPEAN PATENT APPLICATION

(11) **EP 0 667 136 A1**
(43) Date of publication of application: **16.08.1995**
(21) Application number: 95300276.3
(22) Date of filing: 18.01.1995
(51) Int. Cl.: A61F 13/15

(54) **Washable diaper with liquid impervious channel**

(30) Priority: 18.01.1994 US 183288
(71) Applicant: CARING PRODUCTS INTERNATIONAL, INC., New York, New York 10017 (US)
(72) Inventor: Banga, Prakash K., Burnaby, British Columbia (CA)
(74) Representative: Baldock, Sharon Claire

(57) **Abstract**

A washable diaper or incontinence garment is provided having a waterproof interior channel for effectively retaining waste fluids and solids and allowing effective absorption of the waste material in a removable absorbent insert positioned in the channel. In accordance with the invention, the waterproof channel comprises a bottom panel or layer and a pair of longitudinal side walls extending generally upwardly from opposite edges of the bottom panel. The longitudinal side walls extend along the length of the diaper between the front and rear ends. The side walls are spaced-apart to define an opening therebetween for exposing a substantial portion of the surface area of the insert in the channel. This channel design allows easy insertion and removal of the absorbent insert and enables substantial and effective exposure of the pad to the user. In addition, the generally upstanding side wall configuration effectively forms a well for the secure retention of waste in the channel even when the wearer is lying down. The side walls are hemmed along their upper edges with rubber strips under tension.

## Description

The present invention relates to infant diapers and incontinence garments.

One problem with existing disposable infant diapers is that they are costly and wasteful because the entire diaper is discarded when it is soiled or wetted even slightly. Fitted reusable cloth diapers are less popular than disposable diapers because they are generally less effective in retaining waste and require considerable time and labor for washing. Non-fitted cloth diapers are difficult to fit on the infant and also involve considerable labor for washing.

U.S. Patent No. 4,695,279 issued to Steer discloses a reusable diaper for use with a disposable absorbent pad. The pad is retained in a liquid impervious pocket attached in the crotch area of the diaper. The pocket includes a small oval opening across the front face thereof for exposing the pad retained therein. The oval opening is defined by a pair of overlapping flaps at opposite ends of the pocket. This configuration causes a substantial portion of the pad to be sheathed by the pocket. The surface area of the pad exposed to the wearer is thereby reduced, making the pad less effective. In addition, the overlapping flap configuration makes insertion and removal of the pad difficult.

One object of the present invention is to provide infant diapers and incontinence garments that combine the convenience and effectiveness of disposable diapers with the economy and environmental benefits of cloth diapers.

A washable diaper or incontinence garment is provided having a waterproof interior channel for effectively retaining waste fluids and solids and allowing effective absorption of the waste material in a removable absorbent insert positioned in the channel. In accordance with the invention, the waterproof channel comprises a bottom panel or layer and a pair of longitudinal side walls extending generally upwardly from opposite edges of the bottom panel. The longitudinal side walls extend along the length of the diaper between the front and rear ends. The side walls are spaced-apart to define an opening therebetween for exposing a substantial portion of the surface area of the insert in the channel. This channel design allows easy insertion and removal of the absorbent insert and enables substantial and effective exposure of the pad to the user. In addition, the generally upstanding side wall configuration effectively forms a well for the secure retention of waste in the channel even when the wearer is lying down. The side walls are hemmed along their upper edges with rubber strips under tension.

The invention will now be described by way of example with reference to the accompanying drawings in which:
Figure 1 is a perspective view of an infant diaper in accordance with the invention,
Figure 2 is a top view of the channel portion of the diaper with an absorbent insert therein,
Figure 3 is a cross-section view of taken generally along line 3-3 of Figure 2,
Figure 4 is a perspective view of an absorbent insert useable in the diaper,
Figure 5 is a cross-section view of a channel and fabric body similar to Figure 3, showing an alternate, second means of attachment,
Figure 6 is a cross-section view of a channel and fabric body, showing a third means of attachment,
Figure 7 is a cross-section view of a channel and fabric body, showing a fourth means of attachment,
Figure 8 is a cross-section view of a channel and fabric body, showing a fifth means of attachment, and
Figure 9 is a cross-section view of a channel and fabric body, showing a sixth means of attachment.

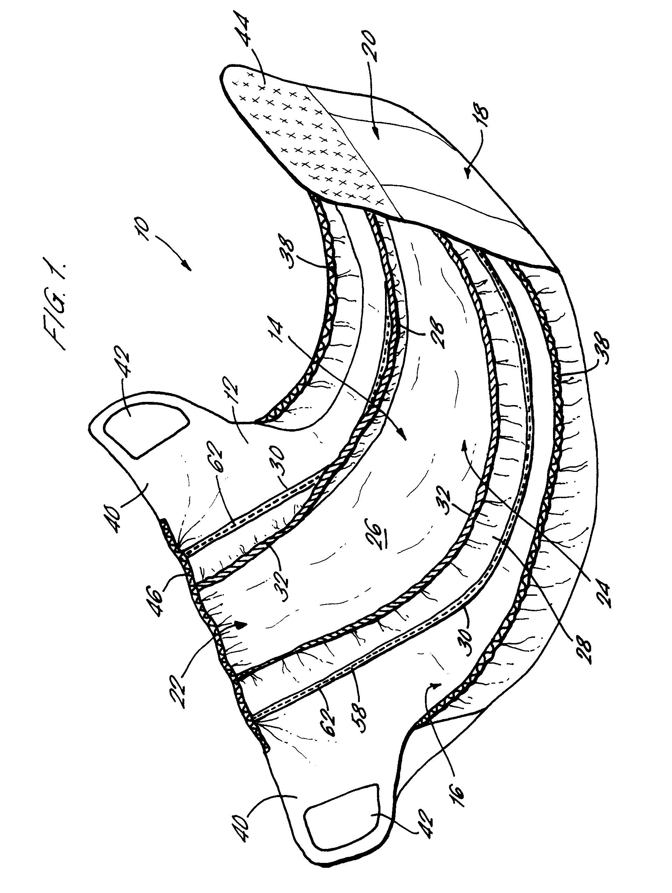

Figure 1 illustrates an infant diaper 10 in accordance with the present invention. The diaper 10 comprises a fabric body or shell 12 and a liquid impervious channel 14 secured therein. The body 12 is constructed of a breathable, polyester fabric that need not be water impervious. The body 12 includes an inner surface 16, an outer surface 18, a front portion 20, a rear portion 22, and a crotch area 24 between the front and rear portions.

The channel 14 is secured to the crotch area 24 of the diaper 10, extending between the front and rear portions. The channel 14 comprises a rectangular bottom panel or layer 26 disposed on the inner surface 16 of the diaper body 12 and a pair of longitudinal side walls 28 extending generally upwardly from the longitudinal edges 30 of the bottom panel 26. The side walls 28 are hemmed along their upper edges 32 with elastic strips under tension.

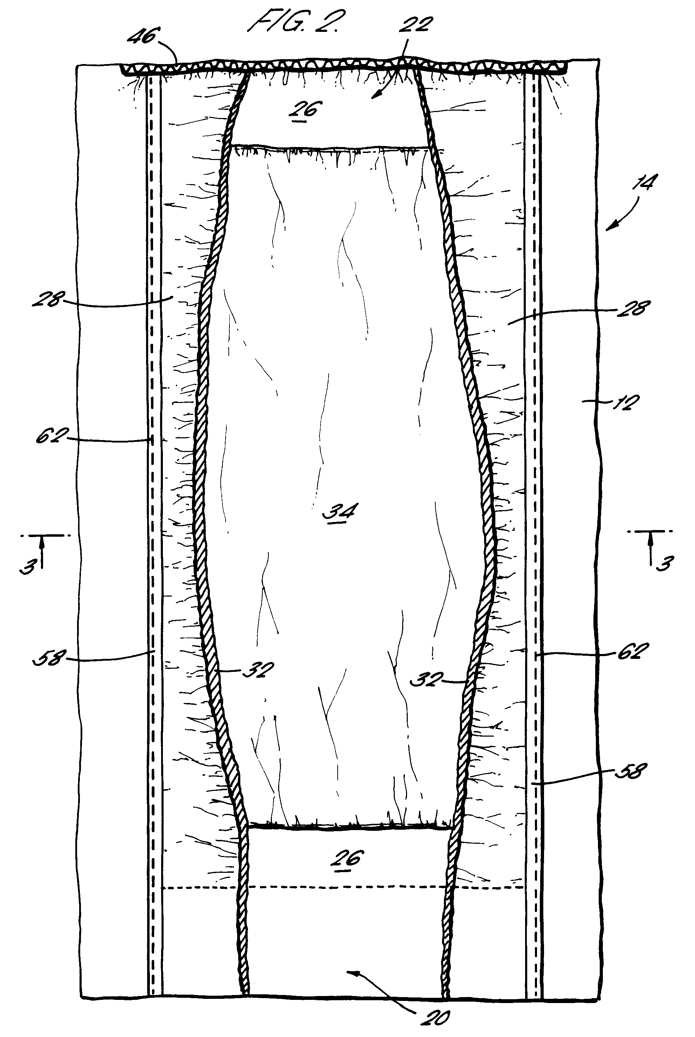

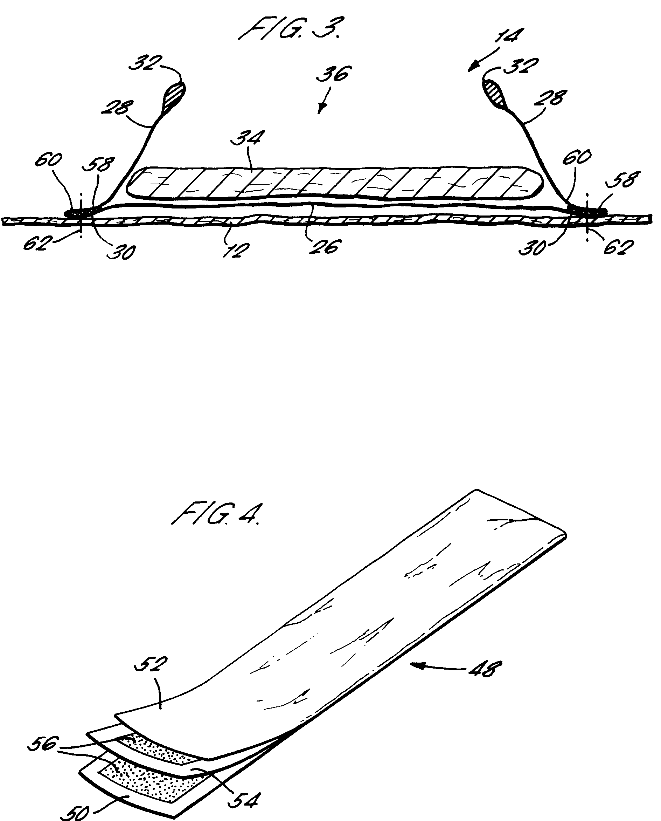

The channel 14 is adapted to receive an absorbent pad or insert 34 therein as shown in Figures 2 and 3. The side walls 28 of the channel 14 extend between the front and rear portions of the diaper 10 in a generally parallel configuration. The side walls 28 are spaced apart to define a significant opening 36 therebetween for exposing a substantial portion of the surface area of the absorbent insert 34 to the diaper wearer.

As shown in Figure 3, the generally upwardly extending side walls 28 effectively form a well that is capable of securely retaining solid and liquid waste (not shown) not absorbed by the pad 34 during use of the diaper 10.

The bottom panel 26 and side walls 28 of the channel 14 are constructed of a man made fabric, like polyester or nylon. The fabric is provided with a waterproof coating of a substance like polyurethane or polyvinyl chloride. The side walls 28 are provided with the waterproof coating on both sides thereof to prevent any wicking of liquid through the hemming at the upper edges 32 of the side walls 28.

As shown in Figure 1, the outer sides 38 of the diaper body 12 are elasticized to form leg cuffs. The rear portion 22 of the diaper body 12 includes a pair of closure tabs 40. The tabs 40 are provided with patches 42 of the hook portion of hook-and-loop fasteners to engage a patch 44 the loop portion on the front portion 20 of the outer surface 18 of the diaper body 12. Alternatively, the hook-and-loop fasteners may be replaced with double hook-and-eye metal fasteners (not shown). Two hooks and two eyes would be used, side by side, to prevent rotation of the fastener about the point of fastening. A metal hook-and-eye fastener has the advantage over hook-and-loop fasteners of being more durable under exposure to extreme heat and to chemicals like chlorine. An elastic strip 46 stitched under tension is provided at the rear end of the diaper body to permit the tabs 40 to be outwardly extended for applying pressure when attached.

The absorbent insert 34 is placed into the channel 14 as shown in Figures 2 and 3 prior to use of the diaper 10. The insert 34 may be reusable and washable or disposable. A disposable insert may be manufactured from a super absorbent polymer, including air-laid thermal-bonded materials of the type available for adult incontinence pads manufactured by Merfin Hygienic Products Ltd. The insert may be not only disposable, but also flushable and bio-degradable. (A "flushable" insert is one that disintegrates in water into particulates that can be handled by municipal sewage systems.) For example, a product marketed under the trademark PRIMA by Johnson & Johnson, Inc. provides a bio-degradable absorbent pad for sanitary napkins and the like composed partly of peat moss. In such an application, an unused absorbent insert is inserted in the channel, and the diaper is then placed on the infant in the usual way. Once the insert has been wetted or soiled, it is removed from the channel and either flushed down the toilet or placed in a waste disposal container. If the diaper body 10 has been wetted or soiled it can be rinsed, and will periodically require washing.

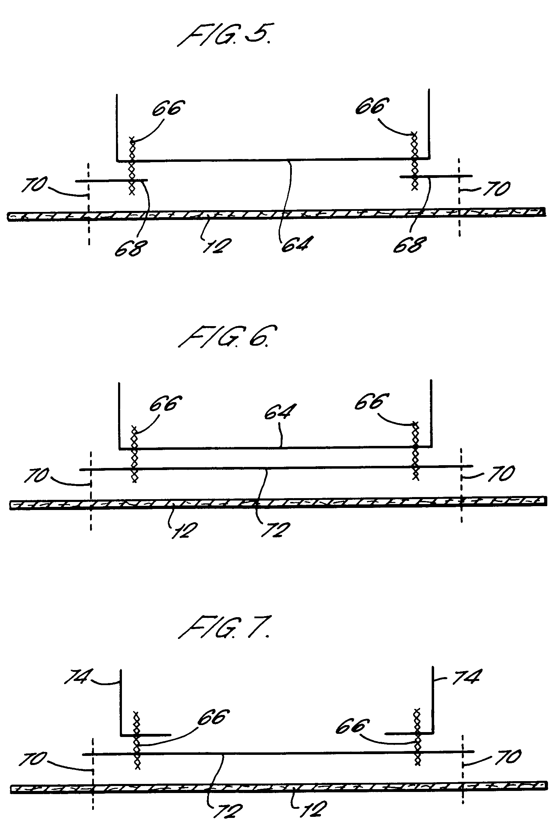

Figure 4 illustrates an absorbent reusable insert 48 that may be used with the diaper 10. The insert 48 is formed of three layers of brushed polyester (two outer layers 50, 52 and a central layer 54), and two layers 56 of absorbent felt. The two felt layers 56 are slightly narrower than the polyester layers 50, 52, 54 and are secured to the central polyester layer 54 by stitching along either longitudinal edge such that the stitching extends through only the felt layers 56 and the central layer 54. The three-piece sandwich thus formed is then, in turn, contained between layers 50, 52 by stitching that extends only through the outer edges of layers 50, 52, 54. This creates a softer outer edge of the insert. Sharp edges are further avoided by staggering the ends of layers 56.

Another type of insert (not shown) that can be used in the diaper 10 comprises a single layer of absorbent felt material covered with a non-wicking "stay dry" material like polyester. The felt layer may be 100% rayon, a blend of rayon and polyester, or a microporous acrylic like that sold under the trademark SUPERSORB. As an alternative to the felt layer, a multiple layer of a cotton fabric, a flannelette, or a bird's eye weave may be used.

Figures 2 and 3 illustrate the attachment of the channel 14 to the diaper body 12. As shown, a pair of flanges 58 are formed by sonic welding the lower ends 60 of the side walls 28 to the adjacent longitudinal edges 30 of the bottom panel 26 along the length of the channel 14. The channel 14 is connected to the diaper body 12 by stitching the flanges 58 to the diaper body 12 along lines 62. This means of attachment permits the channel 14 to be stitched to the diaper body 12 without any stitches extending through the interior of the channel 14, which may affect the water imperviousness of the channel.

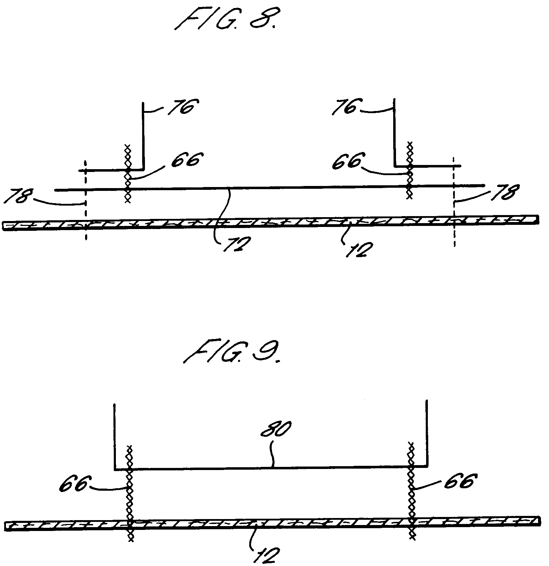

Figures 5 through 9 illustrate other means of attaching channels in accordance with the invention to the fabric body 12. In Figure 5, a channel 64 is sonic welded or glued at seams 66 to two parallel strips 68 made of the same material. The outer edges of strips 68 are then stitched to the underlying fabric body 12 along lines 70. In Figure 6, the strips 68 are replaced with a sheet 72, which extends beyond the edges of the channel 64. In Figure 7, a channel is formed by sonic welding or gluing two L-shaped panels 74 to the bottom sheet 72, with the outer edges of the sheet being stitched to the underlying diaper body 12. In Figure 8, the lower horizontal portions of L-shaped panels 76 extend outwardly to form outer flanges that can be sonic welded along lines 66 to the bottom sheet 72. The flanges can then be stitched to the underlying diaper body 12 along lines 78. A channel 80 may be welded directly to the outer body 12 as shown in Figure 9. However, the outer body should preferably not be made of soft fabrics, which may not be suitable for welding.

The diapers and incontinence garments described above provide a high level of protection from leaks for both active and inactive wearers. The channel secured to the garments enhances the performance of absorbent inserts placed therein by serving as a secure well for moisture not yet absorbed by the insert and as an effective barrier against leaks if the insert has been filled to capacity. The inserts can be easily positioned in the channels without use of pins, adhesive tape, snaps, or other attachment devices.

Another advantage of the channels described above is that the generally upstanding side wall structure and the spaced-apart configuration provides substantial surface area exposure of the absorbent insert to the wearer, thereby increasing the effectiveness of the insert. In addition, the generally upstanding side walls permit easy insertion and removal of the absorbent insert. This is a significant advantage over prior art pocket designs, in which the overlapping flaps defining the pocket have to be physically separated to insert or remove the absorbent insert.

The waterproof channels described above can be attached to the crotch area of other types of garments like underwear, bathing suits and the like for receiving absorbent inserts therein.

The present invention has been described in the foregoing specification with respect to specific embodiments. These embodiments serve as examples to illustrate the invention rather than to limit its scope. Modifications may be made to the illustrated embodiments without departing from the broader teachings of the invention.

## Claims

1. An incontinence garment comprising
A fabric shell that may be removably fitted on a wearer, said fabric shell having an inner surface, an outer surface, a front portion, a rear portion, and a crotch area between the front and rear portions; and
a liquid impervious channel for receiving an absorbent insert therein, said channel being secured to the interior surface of said fabric shell in said crotch area, said channel including a liquid impervious bottom layer having two opposing longitudinal edges extending between the front and rear portions of the shell, and two longitudinal side walls, each extending from one of said longitudinal edges of said bottom layer, said side walls being spaced apart to define an open area therebetween for exposing a substantial portion of the insert to the wearer when the garment is worn.

2. The garment of claim 1, wherein said side walls are generally upstanding with respect to said bottom layer.

3. The garment of claim 1 or claim 2, wherein said side walls include elasticized upper edges.

4. The garment of any preceding claim, wherein said channel comprises a man-made fabric provided with a waterproof coating.

5. The garment of claim 4, wherein said waterproof coating comprises polyurethane and/or polyvinyl chloride.

6. The garment of claim 4 or claim 5, wherein the side walls of said channel are provided with the waterproof coating on both sides thereof.

7. The garment of any preceding claim, wherein said channel further comprises flanges extending along said longitudinal edges and said channel is secured to said inner surface of said fabric shell along said flanges.

8. The garment of claim 7, wherein said flanges are formed by sonic welding lower edges of said side walls adjacent said bottom layer to the longitudinal edges of said bottom layer.

9. The garment of any preceding claim, further comprising the absorbent insert fitted in said channel.

10. The garment of claim 9, wherein said insert comprises two absorbent layers, a central web having an outer edge, and two outer layers, and wherein said two absorbent layers are secured to said central web, and said two outer layers secured to each other and to the outer edge of said central web.

11. The garment of any preceding claim, wherein the fabric shell is not waterproof.

12. An infant diaper, comprising:
a fabric body that may be removably fitted on a wearer, said fabric body having an interior and an exterior surface, a front portion and a rear portion, and a crotch area;
a liquid impervious channel secured to the interior surface in the crotch area of said fabric body, said channel including a generally rectangular shaped liquid impervious bottom layer having four edges, a pair of opposing, generally upstanding liquid impervious side walls extending from two opposite edges of said bottom layer, said channel being positioned in said fabric body such that said two side walls extend between said front and rear portions of said fabric body, said side walls being spaced apart and defining an opening therebetween; and
a removable absorbent pad disposed in said channel wherein a substantial portion of the pad is exposed by said opening to the wearer when the diaper is worn.

13. The diaper of claim 12 having the further features of any one of claims 4, 5, 6 or 11.

14. The diaper of claim 12 or claim 13, wherein said channel further comprises flanges extending along said two opposite edges of said channel and said channel is secured to said inner surface of said fabric body along said flanges.

15. The diaper of claim 14, wherein said flanges are formed by sonic welding lower edges of said side walls adjacent said bottom layer to said opposite two edges of said bottom layer.

16. A non-absorbent, liquid impervious channel for receiving an absorbent pad therein and adapted for being secured to a crotch area of a garment, said channel comprising a generally rectangular shaped liquid impervious bottom layer having four edges, a pair of opposing, substantially upstanding liquid impervious side walls extending from two opposite edges of said bottom layer, said side walls being spaced apart defining an opening therebetween for exposing a surface of the absorbent pad to a wearer of the garment.

17. The channel of claim 16, wherein said side walls include elasticized upper edges.

18. The channel of claim 16 or claim 17, wherein the channel is adapted for being secured to the crotch area of a garment that is not waterproof.
